# EUROPÄISCHE PATENTSCHRIFT

(11) **EP 1 418 433 B1**
(45) Veröffentlichungstag und Bekanntmachung des Hinweises auf die Patenterteilung: **03.01.2007**
(21) Anmeldenummer: 03023863.8
(22) Anmeldetag: 21.10.2003
(51) Int. Cl.: G01N 33/574, C12Q 1/68

(54) **Verfahren zum Nachweis von heptazellulärem Karzinom und Testsystem**
Method for detecting hepatocellular carcinoma and testsystem therefor
Méthode pour détècter du carcinome hépatocellulaire et système d'essai

(30) Priorität: 29.10.2002 DE 10250530
(43) Veröffentlichungstag der Anmeldung: 12.05.2004
(73) Patentinhaber: Mediagnost Gesellschaft für Forschung und Herstellung von Diagnostika GmbH, 72770 Reutlingen (DE)
(72) Erfinder: Haage, Angelika, Dr., 72762 Reutlingen (DE)
(74) Vertreter: Walcher, Armin

(56) Entgegenhaltungen:
- WO-A-02/06484
- WO-A-02/090580
- REEVE J G ET AL: "INSULIN-LIKE GROWTH-FACTOR-BINDING PROTEIN GENE EXPRESSION AND PROTEIN PRODUCTION BY HUMAN TUMOR CELL LINES" INTERNATIONAL JOURNAL OF CANCER, Bd. 51, Nr. 5, 1992, Seiten 818-821, XP008026883 ISSN: 0020-7136
- MOHNIKE KLAUS L ET AL: "Serum levels of insulin-like growth factor-I, -II and insulin-like growth factor binding proteins -2 and -3 in children with acute lymphoblastic leukaemia" EUROPEAN JOURNAL OF PEDIATRICS, Bd. 155, Nr. 2, 1996, Seiten 81-86, XP002268851 ISSN: 0340-6199
- GRAY SG ET AL: "Altered expression of members of the IGF-axis in hepatoblastomas" BRITISCH JOURNAL OF CANCER, Bd. 82, Nr. 9, 2000, Seiten 1561-1567, XP002269145
- AKMAL SN ET AL: "Insulin-like growth factor 2 and insulin-like growth factor binding protein 2 expression in hepatoblastoma" HUMAN PATHOLOGY, Bd. 26, Nr. 8, 1995, Seiten 846-851, XP002269146
- RANKE MICHAEL B ET AL: "Pilot study of elevated levels of insulin-like growth factor-binding protein-2 as indicators of hepatocellular carcinoma." HORMONE RESEARCH (BASEL), Bd. 60, Nr. 4, 2003, Seiten 174-180, XP008026848 ISSN: 0301-0163
- MUELLER HERMANN L ET AL: "Insulin-Like Growth Factor-Binding Protein-2 Concentrations in Cerebrospinal Fluid and Serum of Children with Malignant Solid Tumors or Acute Leukemia" JOURNAL OF CLINICAL ENDOCRINOLOGY AND METABOLISM, Bd. 79, Nr. 2, 1994, Seiten 428-434, XP001184773 ISSN: 0021-972X
- HOLT R I G ET AL: "Gene expression and serum levels of insulin-like growth factors (IGFs) and IGF-binding proteins in a case of non-islet cell tumour hypoglycaemia" GROWTH HORMONE AND IGF RESEARCH, Bd. 8, Nr. 6, Dezember 1998 (1998-12), Seiten 447-454, XP008026882 ISSN: 1096-6374
- MIDORIKAWA YUTAKA ET AL: "Principal component analysis on hepatocellular carcinoma;identification of differently regulated genes in the progression of liver cancer." PROCEEDINGS OF THE AMERICAN ASSOCIATION FOR CANCER RESEARCH ANNUAL, Bd. 44, Juli 2003 (2003-07), Seite 477, XP008026851 94th Annual Meeting of the American Association for Cancer Research;Washington, DC, USA; July 11-14, 2003, July 2003 ISSN: 0197-016X
- MOHNIKE K ET AL: "[Serum concentrations of insulin-like growth factors (IGF)-I and IGF-II and IGF binding proteins (IGFBP)-2 and IGFBP-3 in 49 children with ALL, NHL or solid tumors]" KLINISCHE PADIATRIE. GERMANY 1995 JUL-AUG, Bd. 207, Nr. 4, Juli 1995 (1995-07), Seiten 225-229, XP008027052 ISSN: 0300-8630

## Beschreibung

Die vorliegende Erfindung betrifft ein Verfahren zum Nachweis von hepatozellulärem Karzinom in einem isolierten Probenmaterial und ein Testsystem dafür.

Die rechtzeitige Diagnose von Leberzellkarzinom, insbesondere eines primären Leberzellkarzinoms, ist für den Therapieerfolg einer solchen Erkrankung entscheidend. Die Aussichten einer wirkungsvollen Therapie sind bisher jedoch meist schlecht, da die Diagnose überwiegend zu spät gestellt wird.

Als Diagnoseverfahren werden zur Zeit die Ultraschalldiagnostik, Computertomographie, Laparoskopie mit Biopsie und Leberszintigraphie verwendet. Ein weiteres Indiz für eine maligne Lebererkrankung können erhöhte Serumwerte an alkalischer Phosphatase und das Vorhandensein von α-Fetoprotein sein.

Bei adrenokortikalen Tumoren, akuter Leukämie bei Kindern und Prostatakarzinom wurde festgestellt, daß es zu einem erhöhten Serumpegel des Bindungsproteins-2 der Insulin-ähnlichen Wachstumsfaktoren (IGFBP-2, engl.: Insulin-like-Growth-Factor-Binding Protein-2) kommt. IGFBP-2 gehört zu einer IGFBP-Familie, von der bislang sechs verschiedene Mitglieder identifiziert wurden, die als IGFBP-1 bis IGFBP-6 bezeichnet werden. Die IGFBPs sind Proteine mit etwa 200 - 300 Aminosäuren und weisen ein Molekulargewicht von etwa 24.000 Dalton bis etwa 43.000 Dalton auf. IGFBP-2 ist ein Protein mit einem Molekulargewicht von etwa 32.000 bis etwa 34.000 Dalton.

Die IGFBPs regulieren unter anderem den Einfluß von Insulin ähnlichem Wachstumsfaktor (IGF, engl.: Insulin-like Growth-Factor) auf die Proliferation von Zellen, in dem sie die Zugänglichkeit der IGF-Rezeptoren modulieren. Die IGF I und II scheinen dabei signifikante Effekte auf das Zellwachstum, die Zelldifferenzierung und Zellfunktion im Laufe der prä- und postnatalen Entwicklung auszuüben. Die Wirkung von IGF I und II kann dabei sowohl auf dem endokrinen, dem parakrinen als auch dem autokrinen Weg erfolgen und wird durch den IGF-Typ I-Rezeptor vermittelt, der die IGFs mit hoher Affinität und Spezifität bindet.

Die Bindungsproteine der Insulin-ähnlichen Wachstumsfaktoren (IGFBP) modulieren dabei die Wirkung der IGFs und können dabei inhibierende als auch stimulierende Wirkungen auf Zellen haben (Wex et al., British Journal of Cancer (1998) **78** (4), 515 - 520 : Elevated serum levels of IGFBP-2 found in children suffering from acute leukaemia is accompanied by the occurence of IGFBP-2 mRNA in the tumour clone; Ho Jean P. und Baxter Robert C., Clinical Endocrinology (1997) **46**, 145 - 154: Insulin-like growth-factor-binding protein-2 in patients with prostate carcinoma and benign prostatic hyperplasia; Boulle N. et al., European Journal of Endocrinology (2001) 144 29-36: Evaluation of plasma insulin-like growth factor binding protein-2 as a marker for adrenocortical tumors; Müller Hermann L. Sozialpädiatrie, Kinder- und Jugendheilkunde 6/97, 232 - 233: Insulin-like Growth-Factors und Bindungsproteine: Bedeutung für Tumorzellwachstum in vitro und in vivo).

Im Serum von Patienten mit verschiedenen soliden Tumoren (z.B. Wilms Tumor, Ewing-Sarkom, Neuroblastom) wird eine erhöhte Konzentration des IGFBP-2 Proteins nachgewiesen (Mohnike Klaus et al., Klinische Pädiatrie. 207 (1995) 225-229: Serumkonzentrationen der insulinähnlichen Wachsturnfaktoren (IGF)-I und IGF-II sowie der IGF Bindungsproteine (IGFBP)-2 und IGFBP-3 bei 49 Kindern mit ALL, NHL oder soliden Tumoren). Ferner wird auch bei verschiedenen Tumorzelllinien die erhöhte Produktion von IGFBP im Medium nachgewiesen (Reeve G Julie et al., International Journal of Cancer (1992) 51, 818-821: Insulin-like growth-factor-binding protein gene expression and protein production by human tumour cell lines).

Aufgabe der Erfindung ist es ein Verfahren bereitzustellen, das eine verbesserte und frühzeitigere Diagnose von hepatozellulärem Karzinom ermöglicht.

Noch eine weitere Aufgabe der Erfindung ist es, ein Testsystem zur Diagnose von hepatozellulärem Karzinom bereitzustellen.

Die der Erfindung zugrundeliegende Aufgabe wird durch ein Verfahren zum Nachweis von hepatozellulärem Karzinom in einem isolierten Probenmaterial, umfassend die Schritte
a) Bereitstellen eines isolierten biologischen Probenmaterials,
b) Inkontaktbringen des biologischen Probenmaterials mit einem für IGFBP-2 und/oder für IGFBP-2 kodierende mRNA spezifischen Nachweisreagens,
c) Bestimmen eines Meßwertes, der mit der Menge an IGFBP-2 und/oder der für die IGFBP-2 kodierender mRNA-Menge korreliert,
d) Vergleichen des in Schritt c) bestimmten Meßwertes mit einem gelöst. Referenzwert, wobei das Probenmaterial eine Körperflussigkeit ist,
gelöst.

Bevorzugte Weiterbildungen des erfindungsgemäßen Verfahrens sind in den Ansprüchen 2 bis 12 angegeben.

Die Aufgabe der Erfindung wird weiterhin durch ein Testsystem gemäß Anspruch 13 zum Nachweis von hepatozellulärem Karzinom in einem isolierten Probenmaterial, umfassend eine Festphase mit einer IGFBP-2 oder IGFBP-2 kodierende mRNA bindefähigen Beschichtung und eine Festphase mit einer für einen weiteren hepatozellulären Tumormarker oder mit einer für einen weiteren hepatozellulären Tumormarker kodierende mRNA bindefähigen Beschichtung gelöst.

Bevorzugte Weiterbildungen des erfindungsgemäßen Testsystems sind in den Ansprüchen 14 bis 21 angegeben.

Das erfindungsgemäße Diagnoseverfahren wird nicht am Menschen oder Tier durchgeführt. Das erfindungsgemäße Verfahren wird ausschließlich in vitro, beispielsweise in einem Reaktionsgefäß oder in einer Mikrotiterplatte oder auf einem Biochip, an isoliertem biologischen Probenmaterial von nichtärztlichem Laborpersonal durchgeführt.

Vorzugsweise wird als Probenmaterial eine Körperflüssigkeit verwendet, die vorzugsweise aus der Gruppe ausgewählt wird, die aus Vollblut, Blutplasma, Serum, Cerebrospinalflüssigkeit, Sputum, Urin, Muttermilch, Sperma, Biopsiematerial/-eluat und Gemischen davon besteht.

Das erfindungsgemäße Verfahren kann äußerst vorteilhaft als nicht-invasives Verfahren, beispielsweise an einer Serum- oder Liquor cerebrospinalis-Probe, durchgeführt werden. Mithin ist in diesem Fall beispielsweise keine Leberbiopsie oder Laparoskopie zur Diagnose von Leberzellkarzinom erforderlich.

Es hat sich gezeigt, daß der IGFBP-2-Spiegel im Serum und in der Cerebrospinalflüssigkeit bei einem hepatozellulärem Karzinom, insbesondere bei einem primären hepatozellulären Karzinom, im Vergleich zu den üblicherweise vorliegenden Normalwerten bei nicht erkrankten Menschen, stark erhöht ist.

Vorteilhaft erlaubt die Bestimmung des IGFBP-2-Spiegels in einer Körperflüssigkeit, vorzugsweise im Serum oder in der Cerebrospinalflüssigkeit, eine frühzeitige Diagnose eines Leberzellkarzinoms. Es hat sich herausgestellt, daß der Anstieg des IGFBP-2-Spiegels, d.h. der Konzentration des IGFBP-2 in der untersuchten Körperflüssigkeit, unabhängig vom Alter und dem Ernährungsstatus der untersuchten Person ist. Dabei wurde weiterhin überraschenderweise festgestellt, daß der Spiegel an IGFBP-2 bzw. an für IGFBP-2 kodierende mRNA in der untersuchten Körperflüssigkeit, vorzugsweise Serum oder Cerebrospinalflüssigkeit, ungewöhnlich hoch ist.

Der Spiegel an IGFBP-2 kann dabei das 2- bis 20-fache, üblichweise das 4- bis 15- fache, beispielsweise das 10-fache des Normalwertes an IGFBP-2 in der jeweiligen Körperflüssigkeit, vorzugsweise im Serum oder in der Cerebrospinalflüssigkeit, aufweisen.

Dieser ungewöhnlich hohe Spiegel an IGFBP-2 im Serum ist dabei unabhängig vom Alter des Patienten, unabhängig vom BMI (Body-Mass-Index), von der Tumorgröße oder dem Stadium der hepatischen Zirrhose.

Somit erlaubt die Bestimmung des IGFBP-2-Spiegels, d.h. beispielsweise der Konzentration an IGFBP-2 im Serum und/oder der Cerebrospinalflüssigkeit, die Diagnose eines hepatozellulären Karzinoms bereits in einem sehr frühzeitigen Stadium, da die Tumorgröße kein entscheidender Faktor zu sein scheint.

Die rechtzeitige Exzision des Tumorherdes in diesem frühzeitigen Stadium kann die Heilungsaussichten des Patienten bei Leberzellkarzinom deutlich verbessern.

Gemäß dem erfindungsgemäßen Verfahren ist es möglich, den erhöhten Spiegel an IGFBP-2 auf Proteinebene oder aber auch auf mRNA-Ebene zu bestimmen.

Als Nachweisreagens für das Protein IGFBP-2 kann jeder geeignete Rezeptor mit Spezifität für IGFBP-2 verwendet werden. Vorzugsweise wird der IGFBP-2-Rezeptor aus der Gruppe ausgewählt, die anti-IGFBP-2 monoklonale Antikörper oder anti-IGFBP-2-polyklonale Antikörper umfaßt.

Monoklonale Antikörper sind im Handel unter der Bezeichnung MAB674 und MAB6741 von der Firma R&D Systems, Inc., Minneapolis, USA erhältlich. Polyklonale Antikörper sind im Handel unter der Bezeichnung IGF66 und IGF65 bei der Firma Mediagnost GmbH, Reutlingen, Deutschland erhältlich.

Es ist aber auch möglich, daß als IGFBP-2-Rezeptor eine IGFBP-2-Bindungsdomäne von IGF oder eine synthetische spezifisch IGFBP-2 bindende Struktur, vorzugsweise RNA-Oligonukleotide, verwendet wird. Diese IGFBP-2-Bindungsdomäne kann dabei eine unter Erhalt der Bindungsspezifität für IGFBP-2 modifizierte, beispielsweise stabilisierte, IGFBP-2 Bindungsdomäne von IGF sein. Selbstverständlich ist es auch möglich, die IGFBP-2 Bindungsdomäne von IGF in Form eines Fusionsproteins zu verwenden. In dem Fusionsprotein kann die IGFBP-2 Bindungsdomäne weiter stabilisiert sein.

Das IGFBP-2 kann dabei in biologisch aktiver oder inaktiver Form vorliegen. Bspw. ist es möglich, daß das IGFBP-2 aufgrund von proteolytischer Degradation nicht mehr in aktiver Form vorliegt und fragmentiert ist.

Der Nachweis von hepatozellulären Karzinomen über das erfindungsgemäße Verfahren kann auch über den Nachweis eines erhöhten Spiegels an biologisch inaktivem IGFBP-2 bzw. Fragmenten davon erfolgen.

Vorzugsweise erfolgt die Bestimmung der Menge an IGFBP-2 unter Verwendung immunologischer Nachweisverfahren. Als Nachweisverfahren können bspw. ELISA, RIA, Western-Blot, Western-Liganden-Blot oder andere verwendet werden. Bspw. kann ein für IGFPB-2 spezifischer Antikörper an eine Festphase, bspw. Latexperlen, Sephadexperlen, Magnetperlen, etc. oder an Vertiefungen einer Mikrotiterplatte, beispielsweise einer Kunststoffplatte mit 96 Vertiefungen, oder einen Biochip gekoppelt sein.

Das isolierte biologische Probenmaterial kann dann gegebenenfalls in verdünnter Form mit dem an der Festphase immobilisierten Rezeptor, beispielsweise Antikörper, in Kontakt gebracht und für einen ausreichenden Zeitraum und Bindungsbedingungen, beispielsweise bei 25°C, inkubiert werden, wobei der Rezeptor, beispielsweise ein Antikörper, mit IGFBP-2-Molekülen bindet.

Nach Waschen bspw. mit einer im physiologischen Bereich gepufferten Salzlösung, kann das an dem Antikörper gebundene IGFBP-2 über einen markierten, für IGFBP-2 spezifischen Zweitantikörper nachgewiesen werden. Der Zweitantikörper kann dabei radioaktiv markiert, bspw. iodiert, sein. Selbstverständlich ist es auch möglich, daß der Zweitantikörper mit einem Enzym, bspw. alkalischer Phosphatase oder einer Peroxidase, gekoppelt ist, wobei es nach Zugabe eines Substrates zu einer Farbentwicklung oder zur Chemilumineszens kommt. Derartige Nachweisverfahren für immunologische Testsysteme sind dem Fachmann gut bekannt. Die Mengenbestimmung erfolgt dann durch Vergleich mit einer Testreihe bekannter Konzentrationen an IGFBP-2.

Der Bestimmung der Menge von für IGFBP-2 kodierende mRNA kann bspw. über einen Northern-Blot oder anderweitig geführt werden.

Vorzugsweise erfolgt der Nachweis mRNA über RT-PCR. Dabei wird die mRNA unter Verwendung spezifischer Primer, d.h. an die mRNA spezifisch bindende Oligonukleotide, amplifiziert. Das Amplifikationsprodukt kann dann auf herkömmliche Art und Weise nachgewiesen werden. Bspw. ist es möglich, radioaktive Gruppen oder Fluoreszenzgruppen während der Amplifikation in die amplifizierte cDNA einzubauen und entsprechend nachzuweisen.

Im übrigen kann das aus der DE 197 19 001 bekannte Verfahren zum immunologisch funktionalen Nachweis biologisch aktiver Insulin-like Growth-factor-Bindungproteine oder löslicher Insuline-like Growth-factor-Rezeptoren im Immunoassay verwendet werden.

Dieses Verfahren umfaßt wenigstens zwei Schritte, wobei in einem ersten Schritt das Bindungsprotein aus der Probe mit einem spezifischen Antikörper abgetrennt wird. In einem zweiten Schritt erfolgt dann der funktionale Nachweis des abgetrennten Bindungsproteins durch einen markierten Liganden. Das aus der DE 197 19 001 bekannte Verfahren wird unter Bezug hiermit aufgenommen.

Die Diagnose eines hepatozellulären Karzinoms kann weiter abgesichert werden, wenn neben der Bestimmung des Pegels bzw. der Konzentration an IGFBP-2 eine Bestimmung des Pegels bzw. der Konzentration weiterer hepatozellulärer Tumormarker bzw. der Menge an für den hepatozellulären Tumormarker kodierende mRNA bestimmt wird.

Zur Bestimmung der Menge eines weiteren hepatozellulären Tumormarkers kann jeder geeignete für den hepatozellulären Tumormarker spezifische Rezeptor verwendet werden. Vorzugsweise werden monoklonale Antikörper oder polyklonale Antikörper verwendet.

Als hepatozelluläre Tumormarker werden vorzugsweise α-Fetoprotein, Tissue Polypeptide Antigen, Carcinoembryonales Antigen, Alkalische Phosphatase, Lactat-Dehydrogenase, γ-Glutamyltransferase, Glutamat-Dehydrogenase, Glutamat-Oxalacetat-Transaminase und Mischungen davon verwendet.

Die Mengen dieser weiteren Tumormarker können aus jeder geeigneten Körperflüssigkeit bestimmt werden. Vorzugsweise wird als Körperflüssigkeit Vollblut, Blutplasma, Serum, Sputum, Urin, Muttermilch, Sperma, Biopsiematerial/-eluat, Cerebrospinalflüssigkeit und/oder Gemische davon verwendet.

Der Serumspiegel der vorgenannten Tumormarker ist regelmäßig auch bei hepatozellulären Karzinomen gegenüber den Normalwerten bei gesunden Menschen erhöht.

Die Bestimmung der Menge an IGFBP-2 und die Bestimmung der Menge wenigstens einen der vorgenannten hepatozellulären Tumormarker erhöht die Sicherheit der frühzeitigen Diagnose auf Leberzellkarzinom signifikant.

Äußerst bevorzugt erfolgt die Bestimmung der Menge an IGFBP-2 und einem bzw. mehreren der vorgenannten Tumormarker aus dem selben isolierten Probenmaterial. Bei dieser Vorgehensweise ist ein direkter Vergleich zwischen dem IGFBP-2-Spiegel und dem Spiegel des bzw. der weiter untersuchten Tumormarker möglich.

Ebenso kann die Menge der für einen hepatozellulären Tumormarker kodierenden mRNA in analoger Weise zu der Bestimmung der Menge der für IGFBP-2 kodierenden mRNA unter Verwendung von Nukleotidsonden, d.h. Oligonukleotiden, oder Primern in einer RT-PCR zur Erzeugung von, vorzugsweise markierter, cDNA bestimmt werden.

Die der Erfindung zugrundeliegende Aufgabe wird auch durch ein Testsystem oder ein Testkit mit den Merkmalen des Anspruchs 13 gelöst.

Die in bezug auf das Verfahren vorstehend gemachten Ausführungen gelten entsprechend für das erfindungsgemäße Testsystem.

Vorzugsweise umfaßt die IGFBP-2 bindefähige Beschichtung des Testsystems wenigstens einen IGFBP-2 Rezeptor. Der IGFBP-2-Rezeptor wird bevorzugt aus der Gruppe ausgewählt, die anti-IGFBP-2 monoklonale Antikörper, anti-IGFBP-2 polyklonale Antikörper und Gemische davon umfaßt. Weiterhin ist bevorzugt, daß der IGFBP-2-Rezeptor eine IGFBP-2-Bindungsdomäne von IGF oder eine synthetische spezifisch IGFBP-2 bindende Struktur, vorzugsweise RNA-Oligonukleotide, umfaßt.

Bspw. kann das Testsystem ein Immunoassay, vorzugsweise ein ELISA (engl.: Enzyme-linked Immunosorbent Assay) oder RIA (engl.: Radioimmunoassay) oder Western Blot, sein.

Des weiteren kann auch ein Biochip verwendet werden. Dabei können IGFBP-2 spezifische Antikörper als Proteinarray aufgebracht sein, so daß die gleichzeitige Vermessung einer Vielzahl von Proben verschiedener Patienten bzw. von Proben eines oder mehreren Patienten mit unterschiedlicher Verdünnung erfolgt.

Äußerst vorteilhaft sind auf diesem Biochip bzw. Proteinarray Rezeptoren für IGFBP-2 als auch für weitere Tumormarker, beispielsweise α-Fetoprotein, angeordnet. Als Rezeptoren werden vorzugsweise mono- oder polyklonale Antikörper mit entsprechender Spezifität aufgebracht. Mithin ist die simultane Bestimmung der Menge bzw. Konzentration von IGFBP-2 und weiterer Tumormarker möglich. Da das Probenmaterial für die verschiedenen Mengenbestimmungen aus der selben Probenaufarbeitung und ggf. Probenverdünnung stammt, sind die erhaltenen Meßwerte untereinander direkt vergleichbar und erlauben eine Diagnose von hepatozellulärem Karzinom mit signifikant erhöhter Sicherheit.

Ebenfalls ist es möglich, daß die zum Nachweis von mRNA verwendeten Oligonukleotide in Array-Form angeordnet sind. Die aus der Körperflüssigkeit isolierte mRNA kann in cDNA umgeschrieben werden, wobei während der reversen Transkriptionsreaktion markierte Nukleotide eingebaut werden können. Bspw. Können fluoreszierende Markierungen, beispielsweise Phycoerythrin, oder radioaktive Markierungen, beispielsweise ³²P oder ³³P, verwendet werden.

Auf diese Weise können ebenfalls eine Vielzahl von verschiedenen Proben, bspw. von verschiedenen Patienten oder in verschiedenen Verdünnungen gleichzeitig vermessen werden. Äußerst vorteilhaft kann, wie vorstehend für den Protein-Array beschrieben, simultan eine Mengenbestimmung der Konzentrationen von mRNA betreffend IGFBP-2 als auch weitere Tumormarker, beispielsweise α-Fetoprotein, erfolgen.

Die mit den in Arrayform aufgebrachten Oligonukleotiden bzw. Sonden hybridisierenden Probenmaterialien können dann bspw. unter Verwendung von herkömmlichen Laserscannem oder hochauflösenden CCD-Kameras vermessen werden.

Die Bestimmung der Menge an gebundenem IGFBP-2 bzw. für IGFBP-2 kodierende und hybridisierte mRNA kann dann durch Vergleich der Signalintensität mit entsprechenden Proben bekannter Konzentrationen auf herkömmliche Art und Weise ermittelt werden.

Das erfindungsgemäße Testsystem erlaubt äußerst vorteilhaft die gleichzeitige Bestimmung von IGFBP-2 und von wenigstens einem weiteren hepatozellulären Tumormarker, vorzugsweise α-Fetoprotein, Tissue Polypeptide Antigen, Carcinoembryonales Antigen, Alkalische Phosphatase, Lactat-Dehydrogenase, γ-Glutamyltransferase, Glutamat-Dehydrogenase und/oder Glutamat-Oxalacetat-Transaminase.

Unter Verwendung des erfindungsgemäßen Verfahrens bzw. des erfindungsgemäßen Testsystems wird die Sicherheit der Diagnose auf hepatozelluläres Karzinom in einem isolierten Probenmaterial signifikant verbessert.

### Beispiel

Es standen 50 Patienten zur Verfügung, nämlich 39 männliche und 11 weibliche Patienten, die an hepatozellulärem Karzinom erkrankt waren. Die Diagnose wurde in allen Fällen durch eine histologische Untersuchung bestätigt. Keiner der Patienten bekam, innerhalb der Studiendauer, eine chemotherapeutische Behandlung. Der Ernährungsstatus wurde anhand des Body-Mass-Index [kg/m²] bestimmt. Eine Beschreibung der Ermittlung des Emährungstatus anhand des Body-Mass-Index ist dem Fachmann wohl bekannt.

Die Blutproben wurden am Morgen genommen und bei -20°C gelagert, bis die Proteinbestimmungen durchgeführt wurden. Die Tumorgröße und -anzahl wurde durch sonographische Untersuchungen festgestellt. Die Klassifikation der Tumoren erfolgte nach dem TNM System der Union Internationale Contre le Cancer (UICC). Das TNM-System ist beispielsweise bei Hermanek, P., Scheibe O., Spiessl B. und Wagner G., UICC: TNM-Klassifikation maligner Tumoren, 4. Auflage, 1987, Springer Verlag, Heidelberg, Deutschland, beschrieben.

Die Leberfunktion wurde entsprechend dem Child Pugh - Klassifikationssystem für Leberzirrhose bestimmt. Alle Patienten wurden auf Hepatitis B und C untersucht. Positiv auf Hepatitis C wurden 13 Patienten getestet, während 4 Patienten positiv bzgl. HBCAg getestet wurden.

Als weiterer Tumormarker wurde neben der Bestimmung der Konzentration von IGFBP-2 auch die Konzentration des α-Fetoproteins durchgeführt. Die simultane Bestimmung des α-Fetoproteins veranschaulicht die erhöhte Sicherheit der Diagnose des erfindungsgemäßen Verfahrens. Dem Fachmann ist in Kenntnis der Lehre der vorliegenden Erfindung klar, daß auch einer oder mehrere der oben beispielhaft angegebenen Tumormarker verwendet werden kann.

Die Menge des α-Fetoproteins wurde mit einem Mikropartikel-Enzym-Immunoassay der Fa. Abbott, Wiesbaden, Deutschland durchgeführt. Die obere Grenze der Normalwerte betrug 10,9 ng/ml.

Des weiteren wurden die Serumkonzentrationen von IGF-I, IGF-II, IGFBP-3 durch den Einsatz von Radio-Immunoassays (Firma Mediagnost GmbH, 72770 Reutlingen, Deutschland) bestimmt. Dieser Test ist in Blum W. F., Insulin-like growth factor and their binding proteins, in Ranke M.B. (Hrsg.) Diagnostics of Endocrine Function in Children and Adolescents, Heidelberg-Leipzig, Johann Ambrosius Barth, 1996, Seiten 190-218, beschrieben.

Die Bestimmung der IGFBP-2 Konzentration erfolgte durch einen spezifischen Radio-Immunoassay der Fa. Mediagnost GmbH (s.o.). Dieser Test ist in Blum W.F. et al., Clinical Studies of IGFBP-2 by Radio-Immunoassay, Growth Regul 1993, 3, Seiten 100 - 104, beschrieben.

Als Standard diente rekombinantes humanes IGFBP-2 (Firma Sandoz, Basel, Schweiz). Die Referenzwerte wurden im Rahmen dieser Untersuchung durch die Messung von 427 gesunden Blutspendern im Alter von 17 - 79 Jahren ermittelt.

Um die Daten unabhängig vom Alter auszuwerten, wurden BMI, IGF-I- und IGFBP-Konzentrationen in Standard Deviation Scores (Quotient aus dem gemessenen Wert für den Patienten abzüglich des altersspezifischen, arithmetischen Mittelwertes und der Standardabweichung des arithmetischen Mittels) umgewandelt. Die statistische Analyse erfolgt durch die Bestimmung von Median und Spannbreite. Nach der Methode der kleinsten Quadrate wurde die lineare Regression durchgeführt. Wenn notwendig, wurden die Daten logarithmisch transformiert. Der Vergleich von Datengruppen wurde mittels einer Varianzanalyse (ANOVA: Analysis of Variance) durchgeführt. Signifikante Unterschiede wurden für p≤ 0,01 angenommen. Die Analysen wurden mit der Software Excel 97^{™} durchgeführt.

Die Ergebnisse der Referenzwertbestimmung sind in Tabelle 1 dargestellt. Die einzelnen Daten der Patienten sind in Tabelle 2 aufgeführt.

**Tabelle 1: Altersbezogene Referenzwertbereiche für IGFBP-2 im Serum in [µg/l] von Erwachsenen**

| Alter (Jahre) | Anzahl | Mittel | SD | Median | 5. Perzentile | 95. Perzentile |
|---|---|---|---|---|---|---|
| < 24 | 44 | 319 | 130 | 393 | 35 | 520 |
| 25-34 | 73 | 418 | 190 | 393 | 120 | 751 |
| 35-44 | 99 | 440 | 222 | 395 | 169 | 894 |
| 45-54 | 79 | 410 | 186 | 369 | 160 | 692 |
| 55-64 | 96 | 378 | 159 | 355 | 178 | 688 |
| > 65 | 36 | 445 | 201 | 434 | 182 | 797 |

**Tabelle 2: Ermittelte Patientendaten**

| Tab. 2a: Patientendaten | | | | | | AFP normal, IGFBP-2 normal | | | | |
|---|---|---|---|---|---|---|---|---|---|---|
| Pat.Nr. | Geschlecht | Alter [Jahre] | Zirrhosestadium | Tumor -größe | BMI [kg/m 2] | AFP [kU/L] | IGFBP-2 [µg/L] | IGF-I [µg/L] | IGF-II [µg/L] | IGFBP-3 [µg/L] |
| | | | | | | | | | | |
| 6 | m | 61 | b | 2 | 31 | 12 | 393 | 117 | 476 | 2569 |
| 32 | m | 56 | b | 1 | 26 | 10 | 303 | 35 | 230 | 949 |
| 42 | m | 37 | n.a. | 1 | 25 | 3 | 385 | 9 | 87 | 229 |
| | | | | | | | | | | |

| Tab. 2b: Patientendaten | | | | | | AFP normal, IGFBP-2 hoch | | | | |
|---|---|---|---|---|---|---|---|---|---|---|
| 8 | f | 40 | n.a. | 4 | 19 | 4 | 1418 | n.a. | n.a. | n.a. |
| 13 | m | 82 | n.a. | 4 | n.a. | 2 | 603 | 17 | 156 | 306 |
| 15 | m | 63 | n.a. | 4 | 23 | 8 | 1407 | 9 | 103 | 346 |
| 16 | f | 71 | b | 4 | 27 | 11 | 1073 | n.a. | n.a. | n.a. |
| 17 | m | 80 | n.a. | 1 | 24 | 2 | 629 | 6 | 78 | 221 |
| 34 | m | 55 | b | 4 | 27 | 2 | 7335 | 9 | 75 | 259 |
| 36 | m | 84 | a | 4 | n.a. | 3 | 1590 | 37 | 213 | 637 |
| 38 | m | 70 | a | 4 | 24 | 1 | 1530 | 8 | 167 | 361 |
| 41 | f | 74 | b | 1 | 25 | 9 | 761 | 29 | 230 | 1032 |
| 45 | m | 61 | n.a. | 2 | 23 | 3 | 633 | 38 | 381 | 1562 |
| | | | | | | | | | | |

| Tab. 2c: Patientendaten | | | | | | AFP hoch, IGFBP-2 normal | | | | |
|---|---|---|---|---|---|---|---|---|---|---|
| 21 | m | 69 | b | 4 | 18 | 90 | 647 | 36 | 177 | 999 |
| 24 | m | 59 | n.a. | 4 | 23 | 77 | 381 | 12 | 127 | 394 |
| 39 | m | 71 | n.a. | 1 | 26 | 2780 | 398 | 59 | 363 | 1711 |
| 46 | m | 55 | a | 2 | 25 | 161 | 590 | 4 | 101 | 298 |

| Tab. 2d: Patientendaten | | | | | | AFP hoch, IGFBP-2 hoch | | | | |
|---|---|---|---|---|---|---|---|---|---|---|
| 1 | m | 67 | a | 4 | 23 | 285 | 4740 | 12 | 118 | 414 |
| 2 | m | 73 | c | 4 | n.a. | 134 | 636 | 37 | 286 | 916 |
| 3 | m | 64 | a | 2 | 29 | 149 | 1100 | 19 | 157 | 483 |
| 4 | f | 61 | a | 1 | 17 | 711 | 1008 | n.a. | n.a. | n.a. |
| 5 | m | 62 | a | 4 | 20 | 17000 | 7536 | n.a. | n.a. | n.a. |
| 7 | m | 81 | n.a. | 4 | 23 | 14400 | 1580 | 20 | 186 | 639 |
| 9 | m | 77 | b | 4 | 24 | 1050 | 797 | 37 | 168 | 689 |
| 10 | m | 59 | c | 4 | 28 | 4500 | 932 | 54 | 521 | 2178 |
| 11 | m | 58 | c | 4 | n.a. | 5412 | 799 | 39 | 251 | 1412 |
| 12 | m | 47 | n.a. | 1 | 22 | 13310 | 1196 | 32 | 233 | 750 |
| 14 | m | 67 | c | 4 | 26 | 398 | 2490 | 22 | 153 | 622 |
| 18 | m | 53 | b | 4 | 30 | 15 | 2152 | 33 | 252 | 883 |
| 19 | f | 78 | n.a. | 3 | 19 | 40 | 2024 | 12 | 118 | 163 |
| 20 | m | 66 | c | 4 | 25 | 10000 | 750 | 94 | 479 | 2220 |
| 22 | f | 71 | b | 1 | 31 | 764 | 1120 | 53 | 387 | 1907 |
| 23 | m | 67 | c | 4 | 27 | 807 | 2291 | 3 | 51 | 140 |
| 25 | m | 63 | c | 4 | 28 | 80 | 895 | n.a. | n.a. | n.a. |
| 26 | f | 79 | b | 4 | 23 | 49500 | 1195 | 154 | 377 | 2402 |
| 27 | m | 57 | a | 3 | 18 | 20 | 2177 | n.a. | n.a. | n.a. |
| 28 | m | 66 | b | 1 | 26 | 48 | 344 | n.a. | n.a. | n.a. |
| 29 | f | 79 | a | 3 | 25 | 17000 | 1501 | n.a. | n.a. | n.a. |
| 30 | m | 62 | b | 4 | 29 | 520 | 2558 | 23 | 277 | 967 |
| 31 | m | 75 | n.a. | n.a. | 27 | 150 | 645 | 27 | 151 | 554 |
| 33 | m | 65 | b | 4 | 25 | 1050 | 8042 | n.a. | n.a. | n.a. |
| 35 | m | 77 | a | 1 | 21 | 14 | 927 | 9 | 109 | 399 |
| 37 | m | 61 | c | 4 | 26 | 190 | 1243 | 72 | 502 | 1832 |
| 40 | f | 76 | c | 4 | 16 | 14 | 4692 | 8 | 88 | 330 |
| 43 | f | 74 | n.a. | 3 | 20 | 54 | 2193 | 95 | 598 | 3038 |
| 44 | m | 58 | b | 4 | 30 | 2100 | 1834 | 23 | 161 | 553 |
| 47 | m | 79 | a | 3 | 26 | 3416 | 1700 | 16 | 282 | 575 |
| 48 | f | 53 | c | 4 | .25 | 4000 | 2272 | 36 | 256 | 938 |
| 49 | m | 60 | n.a. | 3 | 28 | 165 | 841 | 25 | 452 | 1802 |
| 50 | m | 73 | b | 4 | 35 | 43 | 2379 | 18 | 161 | 509 |

| | | | | | | | | | | |
|---|---|---|---|---|---|---|---|---|---|---|
| n.a. = nicht verfügbar | | | | | | | | | | |

Zusammenfassend wird der Median und die Spannbreite in Klammern des Patientenkollektivs dargestellt. Der Altersmedian beträgt 66 Jahre (37-84), der Body-Mass Index betrug im Median 25 (34,8-16,4 [kg/m²]). Bei 14 Patienten wurde eine zirrhotische Leber diagnostiziert. Bei 11, 15 und 10 Patienten wurden die Child Pugh Stadien A, B bzw. C beobachtet. Bei 11, 4 und 6 Patienten wurden die Tumorstadien 1, 2 bzw. 3 nachgewiesen. Bei den restlichen 29 Patienten befand sich der Tumor im Stadium 4.

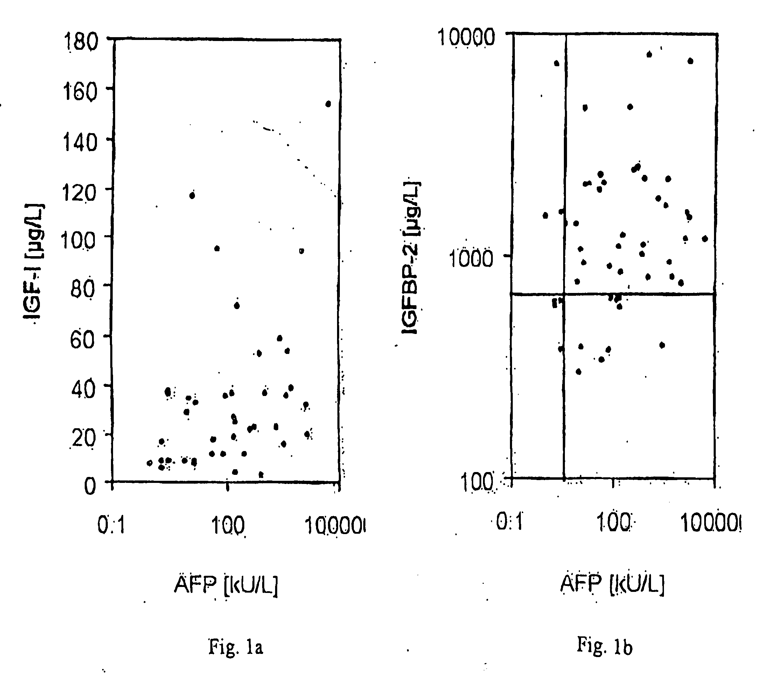

Bei 40 der untersuchten 50 Patienten waren die α-Fetoproteinwerte erhöht (vgl. Fig. 1), die jedoch nicht mit dem BMI, der Tumorgröße, dem Zirrhosegrad oder den IGF-Werten korrelierten.

Im Vergleich zu dem gesunden Kontrollkollektiv waren die IGF-I, IGF-II und IGFBP-3 Konzentrationen signifikant geringer. Die Medianwerte [µg/ml] betrugen 25 für IGF-I, 186 für IGF-II und 639 für IGFBP-3.

Die IGFBP-2 Konzentration überschritt in 37 von 50 Fällen den Normalwert. Wie zu erkennen ist, gibt es keine Abhängigkeit der IGFBP-2 Konzentrationen von der Tumorgröße oder dem Zirrhosestadium. Die überraschende Unabhängigkeit der IGFBP-2 Konzentration, insbesondere im Serum oder in der Cerebrospinalflüssigkeit, von der Tumorgröße oder dem Tumorstadium macht IGFBP-2 zu einem äußerst sensitiven Marker für hepatozelluläres Karzinom. In Kombination mit der Bestimmung der Konzentration von weiteren Tumormarkern für Leberkarzinom, beispielsweise α-Fetoprotein, ermöglicht das erfindungsgemäße Verfahren bzw. das erfindungsgemäße Testsystem ein Diagnose von Leberzellkarzinom mit signifikant verbesserter Diagnosesicherheit.

Die beigefügten Figuren veranschaulichen weiter die Signifikanz eines erhöhten IGFBP-2 Serumspiegels bei der Diagnose von hepatozellulärem Leberzellkarzinom. In sämtlichen Figuren sind die angegebenen Werte bei an hepatozellulärem Leberzellkarzinom erkrankten Patienten dargestellt.

Fig. 1 a zeigt, daß der Serumspiegel von α-Fetoprotein (AFP) gegenüber dem Serumspiegel von IGF signifikant erhöht ist.
Fig. 1 b zeigt, daß sowohl der Serumspiegel von α-Fetoprotein (AFP) als auch von IGFBP-2 signifikant erhöht ist.

Fig. 2a zeigt die Beziehung zwischen Serumspiegeln von IGF-I und Alter.
Fig. 2b zeigt die Beziehung zwischen Serumspiegeln von IGF-I und Body-Mass-Index (BMI).
Fig. 2c zeigt die Beziehung zwischen Serumspiegeln von IGFBP-2 und Alter.
Fig. 2d zeigt die Beziehung zwischen Serumspiegeln von IGFBP-2 und BMI.

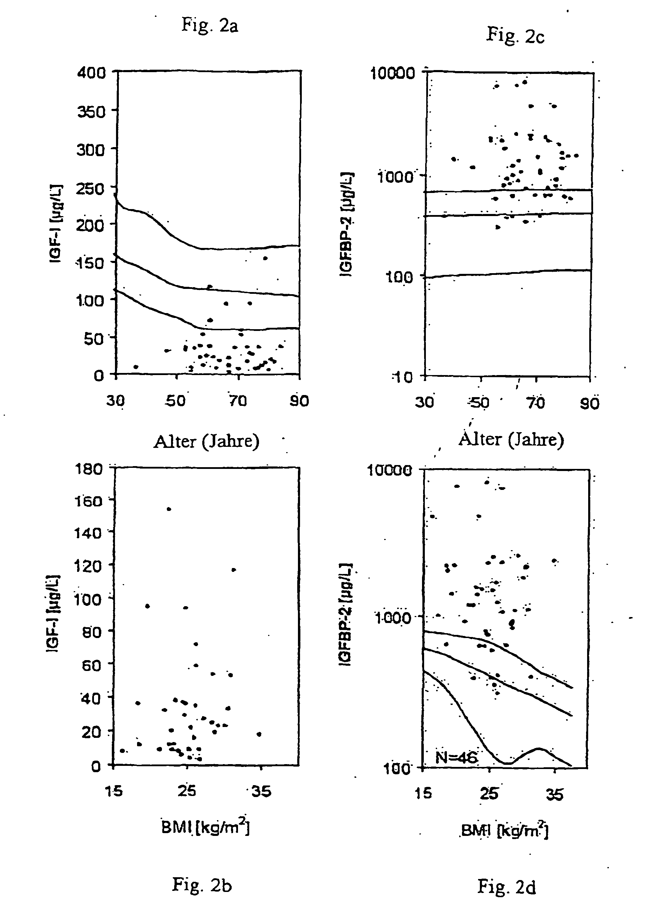

In den Fig. 2a-2d sind die jeweiligen Referenzwerte der 5., 50. und 95. Perzentile angegeben.

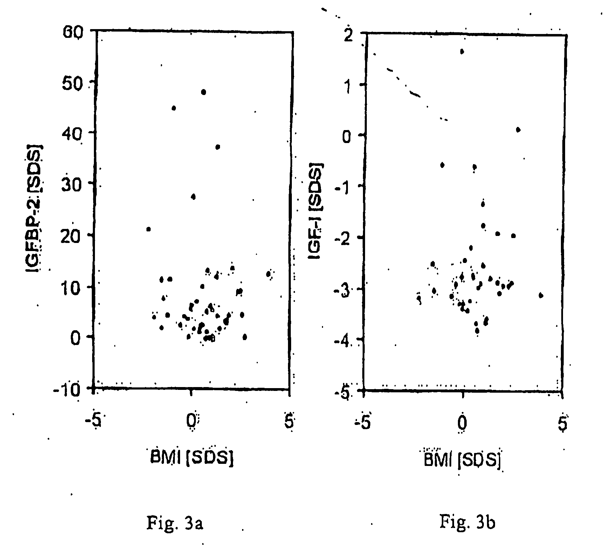

Fig. 3a zeigt die Beziehung zwischen IGFBP-2 (SDS) und BMI (SDS).
Fig. 3b zeigt die Beziehung zwischen IGF-I (SDS) und BMI (SDS).

## Patentansprüche

1. Verfahren zum Nachweis von hepatozellulärem Karzinom in einem isolierten Probenmaterial,
umfassend die Schritte:
(a) Bereitstellen eines isolierten biologischen Probenmaterials,
(b) Inkontaktbringen des biologischen Probenmaterials mit einem für IGFBP-2 und/oder für IGFBP-2 kodierende mRNA spezifischen Nachweisreagenz,
(c) Bestimmen eines Meßwertes, der mit der Menge an IGFBP-2 und/oder der für IGFBP-2 kodierende mRNA-Menge korreliert,
(d) Vergleichen des in Schritt (c) bestimmten Meßwertes mit einem Referenzwert,
wobei das Probenmaterial eine Körperflüssigkeit ist.

2. Verfahren gemäß Anspruch 1,
**dadurch gekennzeichnet,**
**daß** die Körperflüssigkeit aus der Gruppe ausgewählt wird, die aus Vollblut, Blutplasma, Serum. Cerebrospinalflüssigkeit, Sputum, Urin. Muttermilch, Sperma und Gemischen davon besteht.

3. Verfahren nach einem der vorstehenden Ansprüche,
**dadurch gekennzeichnet,**
**daß** das Nachweisreagenz für die für IGFBP-2 kodierende mRNA eine Nukleinsäuresonde ist oder Primer sind.

4. Verfahren nach einem der vorstehenden Ansprüche,
**dadurch gekennzeichnet,**
**daß** das Nachweisreagenz für IGFBP-2 ein IGFBP-2 Rezeptor ist.

5. Verfahren gemäß Anspruch 4,
**dadurch gekennzeichnet,**
**daß** der IGFBP-2-Rezeptor aus der Gruppe ausgewählt wird, die anti-IGFBP-2 monoklonale Antikörper, anti-IGFBP-2 polyklonale Antikörper und Gemische davon umfaßt.

6. Verfahren gemäß Anspruch 4,
**dadurch gekennzeichnet,**
**daß** der IGFBP-2-Rezeptor eine IGFBP-2-Bindungsdomäne von IGF oder eine synthetische spezifisch IGFBP-2 bindende Struktur, vorzugsweise RNA-Oligonukleotide, umfaßt.

7. Verfahren nach einem der vorstehenden Ansprüche,
**dadurch gekennzeichnet,**
**daß** das IGFBP-2 in biologisch aktiver oder inaktiver Form vorliegt.

8. Verfahren gemäß Anspruch 7.
**dadurch gekennzeichnet,**
**daß** die biologisch inaktive Form Fragmente von IGFBP-2 umfaßt.

9. Verfahren nach einem der vorstehenden Ansprüche,
**dadurch gekennzeichnet,**
**daß** die Bestimmung der Menge an IGFBP-2 unter Verwendung immunologischer Nachweisverfahren erfolgt.

10. Verfahren nach einem der vorstehenden Ansprüche,
**dadurch gekennzeichnet**
**daß** das Verfahren weiterhin die folgenden Schritte umfaßt :
e) Inkontaktbringen des biologischen Probenmaterials mit einem für einen weiteren hepatozellularen Tumormarker und/oder eine für einen weiteren hepatozellulären Tumormarker kodierende mRNA spezifischen Nachweisreagenz,
(f) Bestimmen eines Meßwertes, der mit der Menge des hepatozellulären Tumormarkers und/oder der für den hepatozellulären Tumormarker kodierende mRNA-Menge korreliert,
(g) Vergleichen des in Schritt (f) bestimmten Meßwertes mit einem Referenzwert.

11. Verfahren nach Anspruch 10,
**dadurch gekennzeichnet,**
**daß** der Rezeptor für den hepatozellulären Tumormarker aus der Gruppe ausgewählt wird, die monoklonale Antikörper, polyklonale Antikörper und Gemische davon, wobei der oder die Antikörper spezifisch für den hepatozellulären Tumormarker sind, umfaßt.

12. Verfahren nach Anspruch 10 oder 11,
**dadurch gekennzeichnet,**
**daß** der hepatozelluläre Tumormarker aus der Gruppe ausgewählt wird, die aus α-Fetoprotein, Tissue Polypeptide Antigen, Carcinoembryonales Antigen, Alkalische Phosphatase, Lactat-Dehydrogenase, γ-Glutamyltransferase, Glutamat-Dehydrogenase, Glutamat-Oxalacetat-Transaminase und Mischungen davon besteht.

13. Testsystem zum Nachweis von hepatozellulärem Karzinom in einem isolierten Probenmaterial, umfassend
eine Festphase mit einer für IGFBP-2 oder für IGFBP-2 kodierende mRNA bindefähigen Beschichtung und
eine Festphase mit einer für einen weiteren hepatozellulären Tumormarker oder mit einer für einen weiteren hepatozellulären Tumormarker kodierende mRNA bindefähigen Beschichtung, wobei der hepatozelluläre Tumormarker aus der Gruppe ausgewählt wird, die aus α-Fetoprotein. Tissue Polypeptide Antigen, Carcinoambryonales Antigen, alkalische Phosphatase, Lactat-Dehydrogenase, γ-Glutamyltransferase, Glutamat-Dehydrogenase, Glutamat-Oxalacetat-Transaminase und Mischungen davon, besteht.

14. Testsystem nach Anspruch 13,
**dadurch gekennzeichnet,**
**daß** die IGFBP-2 bindefähige Beschichtung wenigstens einen IGFBP-2 . Rezeptor umfaßt.

15. Testsystem nach Anspruch 14,
**dadurch gekennzeichnet,**
**daß** der IGFBP-2-Rezeptor aus der Gruppe ausgewählt wird, die anti-IGFBP-2 monoklonale Antikörper, anti-IGFBP-2 polyklonale Antikörper und Gemische davon umfaßt.

16. Testsystem nach Anspruch 14,
**dadurch gekennzeichnet,**
**daß** der IGFBP-2-Rezeptor eine IGFBP-2-Bindungsdomäne von IGF oder eine synthetische spezifisch IGFBP-2 bindende Struktur, vorzugsweise RNA-Oligonukleotide, umfaßt.

17. Testsystem nach einem der Ansprüche 13 bis 16,
**dadurch gekennzeichnet,**
**daß** die bindefähige Beschichtung einen Rezeptor für den weiteren hepatozellulären Tumormarker umfaßt.

18. Testsystem nach Anspruch 17,
**dadurch gekennzeichnet,**
**daß** der Rezeptor für den hepatozellulären Tumormarker aus der Gruppe ausgewählt wird, die monoklonale Antikörper, polyklonale Antikörper und Gemische davon, wobei der oder die Antikörper spezifisch für den hepatozellulären Tumormarker ist bzw. sind, umfaßt.

19. Testsystem nach einem der Ansprüche 13 bis 18,
**dadurch gekennzeichnet,**
**daß** das Testsystem ein Immunoassay, vorzugsweise ELISA, RIA, Western Blot, oder Biochip sowohl zum spezifischen Nachweis von IGFBP-2 als auch von dem weiteren hepatozellulären Tumormarker bzw. jeweiligen Fragmenten davon ist.

20. Testsystem nach Anspruch 13,
**dadurch gekennzeichnet,**
**daß** die für IGFBP-2 oder den weiteren hepatozellulären Tumormarker kodierende mRNA bindefähige Beschichtung spezifische Nukleinsäuresonden oder Primer aufweist.

21. Testsystem nach Anspruch 20.
**dadurch gekennzeichnet**
**daß** das Testsystem eine RT-PCR, ein Northern Blot oder Biochip sowohl zum spezifischen Nachweis von für IGFBP-2 als auch von für den weiteren hepatozellulären Tumormarker kodierende mRNA oder Fragmenten davon ist.

## Claims

1. Method for detecting hepatocellular carcinoma in an isolated sample material, including the steps:
(a) provision of an isolated biological sample material,
(b) contacting the biological sample material with a detection reagent specific for IGFBP-2 and/or mRNA coding for IGFBP-2,
(c) determination of a measurement which correlates with the amount of IGFBP-2 and/or the amount of mRNA coding for IGFBP-2,
(d) comparison of the measurement determined in step (c) with a reference value,
where the sample material is a body fluid.

2. Method according to Claim 1, **characterized in that** the body fluid is selected from the group which consists of whole blood, blood plasma, serum, cerebrospinal fluid, sputum, urine, breast milk, sperm and mixtures thereof.

3. Method according to any of the preceding claims, **characterized in that** the detection reagent for the mRNA coding for IGFBP-2 is a nucleic acid probe or are primers.

4. Method according to any of the preceding claims, **characterized in that** the detection reagent for IGFBP-2 is an IGFBP-2 receptor.

5. Method according to Claim 4, **characterized in that** the IGFBP-2 receptor is selected from the group which includes anti-IGFBP-2 monoclonal antibodies, anti-IGFBP-2 polyclonal antibodies and mixtures thereof.

6. Method according to Claim 4, **characterized in that** the IGFBP-2 receptor includes an IGFBP-2 binding domain of IGF or a synthetic specifically IGFBP-2 binding structure, preferably RNA oligonucleotides.

7. Method according to any of the preceding claims, **characterized in that** the IGFBP-2 is present in biologically active or inactive form.

8. Method according to Claim 7, **characterized in that** the biologically inactive form includes fragments of IGFBP-2.

9. Method according to any of the preceding claims, **characterized in that** the determination of the amount of IGFBP-2 takes place by using immunological detection methods.

10. Method according to any of the preceding claims, **characterized in that** the method additionally includes the following steps:
(e) contacting the biological sample material with a specific detection reagent for a further hepatocellular tumour marker and/or an mRNA coding for a further hepatocellular tumour marker,
(f) determination of a measurement which correlates with the amount of the hepatocellular tumour marker and/or the amount of mRNA coding for the hepatocellular tumour marker,
(g) comparison of the measurement determined in step (f) with a reference value.

11. Method according to Claim 10, **characterized in that** the receptor for the hepatocellular tumour marker is selected from the group which includes monoclonal antibodies, polyclonal antibodies and mixtures thereof, where the antibody(s) are specific for the hepatocellular tumour marker.

12. Method according to Claim 10 or 11, **characterized in that** the hepatocellular tumour marker is selected from the group which consists of α-fetoprotein, tissue polypeptide antigen, carcinoembryonic antigen, alkaline phosphatase, lactate dehydrogenase, γ-glutamyltransferase, glutamate dehydrogenase, glutamate-oxalacetate transaminase and mixtures thereof.

13. Test system for detecting hepatocellular carcinoma in an isolated sample material, including
a solid phase with a coating capable of binding for IGFBP-2 or mRNA coding for IGFBP-2, and
a solid phase with a coating capable of binding for a further hepatocellular tumour marker or with an mRNA coding for a further hepatocellular tumour marker, where the hepatocellular tumour marker is selected from the group which consists of α-feto-protein, tissue polypeptide antigen; carcinoembryonic antigen, alkaline phosphatase, lactate dehydrogenase, γ-glutamyltransferase, glutamate dehydrogenase, glutamate-oxalacetate transaminase and mixtures thereof.

14. Test system according to Claim 13, **characterized in that** the coating capable of binding IGFBP-2 includes at least one IGFBP-2 receptor.

15. Test system according to Claim 14, **characterized in that** the IGFBP-2 receptor is selected from the group which includes anti-IGFBP-2 monoclonal antibodies, anti-IGFBP-2 polyclonal antibodies and mixtures thereof.

16. Test system according to Claim 14, **characterized in that** the IGFBP-2 receptor includes an IGFBP-2 binding domain of IGF or a synthetic specifically IGFBP-2 binding structure, preferably RNA oligonucleotides.

17. Test system according to any of Claims 13 to 16, **characterized in that** the coating capable of binding includes a receptor for the further hepatocellular tumour marker.

18. Test system according to Claim 17, **characterized in that** the receptor for the hepatocellular tumour marker is selected from the group which includes monoclonal antibodies, polyclonal antibodies and mixtures thereof, where the antibody(s) is or are specific for the hepatocellular tumour marker.

19. Test system according to any of Claims 13 to 18, **characterized in that** the test system is an immuno assay, preferably ELISA, RIA, Western blot, or biochip both for specific detection of IGFBP-2 and of the further hepatocellular tumour marker or respective fragments thereof.

20. Test system according to Claim 13, **characterized in that** the coating capable of binding mRNA coding for IGFBP-2 or the further hepatocellular tumour marker comprises specific nucleic acid probes or primers.

21. Test system according to Claim 20, **characterized in that** the test system is an RT-PCR, a Northern blot or biochip both for specific detection of mRNA coding for IGFBP-2 and of mRNA coding for the further hepatocellular tumour marker, or fragments thereof.

## Revendications

1. Procédé de détermination de carcinome hépatocellulaire dans un matériau échantillon isolé, comprenant les étapes consistant à :
(a) obtenir un matériau échantillon biologique isolé,
(b) mettre le matériau échantillon biologique en contact avec un réactif de détection spécifique à l'IGFBP-2 et/ou à l'ARNm codant pour l'IGFBP-2,
(c) Déterminer une valeur de mesure corrélée avec la quantité d'IGFBP-2 et/ou la quantité d'ARNm codant pour l'IGFBP-2,
(d) comparer la valeur de mesure déterminée à l'étape (c) à une valeur de référence,
dans lequel le matériau échantillon est un fluide corporel.

2. Procédé selon la revendication 1, **caractérisé en ce que** le fluide corporel est choisi dans le groupe constitué par le sang total, le plasma sanguin, le sérum, le liquide cérébrospinal, le crachat, l'urine, le lait maternel, le sperme et des mélanges de ceux-ci.

3. Procédé selon l'une des revendications précédentes, **caractérisé en ce que** le réactif de détection de l'ARNm codant pour l'IGFBP-2 est une sonde d'acide nucléique ou des amorces.

4. Procédé selon l'une des revendications précédentes, **caractérisé en ce que** le réactif de détection de l'IGFBP-2 est un récepteur de l'IGFBP-2.

5. Procédé selon la revendication 4, **caractérisé en ce que** le récepteur de l'IGFBP-2 est choisi dans le groupe comprenant des anticorps anti-IGFBP-2, des anticorps polyclonaux anti-IGFBP-2 et des mélanges de ceux-ci.

6. Procédé selon la revendication 4, **caractérisé en ce que** le récepteur de l'IGFBP-2 comprend un domaine de liaison à l'IGFBP-2 de l'IGF ou une structure synthétique qui se lie spécifiquement à l'IGFBP-2, de préférence des oligonucléotides d'ARN.

7. Procédé selon l'une des revendications précédentes, **caractérisé en ce que** l'IGFBP-2 est présent sous une forme biologiquement active ou inactive.

8. Procédé selon la revendication 7, **caractérisé en ce que** la forme biologiquement inactive comprend des fragments de l'IGFBP-2

9. Procédé selon l'une des revendications précédentes, **caractérisé en ce que** la détermination de la quantité d'IGFBP-2 se fait en utilisant des procédés de détection immunologiques.

10. Procédé selon l'une des revendications précédentes, **caractérisé en ce que** le procédé comprend en plus les étapes suivantes consistant à :
(e) Mettre le matériau échantillon biologique en contact avec un réactif de détection spécifique à un autre marqueur de tumeur hépatocellulaire et/ou à un ARNm codant pour un autre marqueur de tumeur hépatocellulaire,
(f) Déterminer une valeur de mesure corrélée avec la quantité du marqueur de tumeur hépatocellulaire et/ou avec la quantité d'ARNm codant pour un autre marqueur de tumeur hépatocellulaire,
(g) Comparer la valeur de mesure déterminée à l'étape (f) à une valeur de référence.

11. Procédé selon la revendication 10, **caractérisé en ce que** le récepteur du marqueur de tumeur hépatocellulaire est choisi dans le groupe comprenant des anticorps monoclonaux, des anticorps polyclonaux et des mélanges de ceux-ci, le ou lesdits anticorps étant spécifiques au marqueur de tumeur hépatocellulaire.

12. Procédé selon la revendication 10 ou la revendication 11, **caractérisé en ce que** le marqueur de tumeur hépatocellulaire est choisi dans le groupe comprenant l'α-foetoprotéine, l'antigène tissulaire polypeptidique, l'antigène carcino-embryonnaire, la phosphatase alcaline, la lactate déshydrogénase, la γ-glutamyltransférase, la glutamate déshydrogénase, la glutamate oxalacétate transaminase et des mélanges de ceux-ci.

13. Système de test pour la détermination de carcinome hépatocellulaire dans un matériau échantillon isolé, comprenant une phase solide avec un revêtement capable de se lier à l'IGFBP-2 ou à l'ARNm codant pour l'IGFBP-2 et une phase solide avec un revêtement capable de se lier à un autre marqueur de tumeur hépatocellulaire ou à un ARNm codant pour un autre marqueur de tumeur hépatocellulaire, le marqueur de tumeur hépatocellulaire étant choisi dans le groupe constitué par l'α-foetoprotéine, l'antigène tissulaire polypeptidique, l'antigène carcino-embryonnaire, la phosphatase alcaline, la lactate déshydrogénase, la γ-glutamyltransférase, la glutamate déshydrogénase, la glutamate oxalacétate transaminase et des mélanges de ceux-ci.

14. Système de test selon la revendication 13, **caractérisé en ce que** le revêtement capable de lier à l'IGFBP-2 comprend au moins un récepteur de l'IGFBP-2.

15. Système de test selon la revendication 14, **caractérisé en ce que** le récepteur de l'IGFBP-2 est choisi dans le groupe comprenant des anticorps monoclonaux, des anticorps polyclonaux et des mélanges de ceux-ci.

16. Système de test selon la revendication 14, **caractérisé en ce que** le récepteur de l'IGFBP-2 comprend un domaine de liaison à l'IGFBP-2 de l'IGF ou une structure synthétique qui se lie spécifiquement à l'IGFBP-2, de préférence des oligonucléotides d'ARN.

17. Système de test selon l'une des revendications 13 à 16, **caractérisé en ce que** le revêtement capable de se lier comprend un récepteur de l'autre marqueur de tumeur hépatocellulaire.

18. Système de test selon la revendication 17, **caractérisé en ce que** le récepteur du marqueur de tumeur hépatocellulaire est choisi dans le groupe comprenant des anticorps monoclonaux, des anticorps polyclonaux et des mélanges de ceux-ci, le ou lesdits anticorps étant spécifiques au marqueur de tumeur hépatocellulaire.

19. Système de test selon l'une des revendications 13 à 18, **caractérisé en ce que** le système de test est un immunodosage, de préférence un ELISA, un RIA, un Western Blot, ou une biopuce pour la détection spécifique tant de l'IGFBP-2 que de l'autre marqueur de tumeur hépatocellulaire ou de leurs fragments respectifs.

20. Système de test selon la revendication 13, **caractérisé en ce que** le revêtement capable de se lier à l'ARNm codant pour l'IGFBP-2 ou à l'ARNm codant pour l'autre marqueur de tumeur hépatocellulaire présente des sondes d'acides nucléiques ou des amorces spécifiques.

21. Système de test selon la revendication 20, **caractérisé en ce que** le système de test est une RT-PCR, un Northern Blot ou une biopuce pour la détection spécifique tant de l'ARNm codant pour l'IGFBP-2 que de l'ARNm codant pour l'autre marqueur de tumeur hépatocellulaire, ou de leurs fragments.
